# EUROPEAN PATENT SPECIFICATION

(11) **EP 2 281 575 B1**
(45) Date of publication and mention of the grant of the patent: **07.08.2013**
(21) Application number: 09731296.1
(22) Date of filing: 08.04.2009
(51) Int. Cl.: A61K 39/395, A61P 35/00, C07K 16/18, C07K 16/30

(54) **ANTIBODIES FOR TREATING CANCER**
ANTIKÖRPER ZUR BEHANDLUNG VON KREBS
ANTICORPS DESTINÉS AU TRAITEMENT DU CANCER

(30) Priority: 09.04.2008 ES 200801071
(43) Date of publication of application: 09.02.2011
(73) Proprietor: Universidad de Barcelona, 08028 Barcelona (ES); Hospital Clinic i Provincial de Barcelona, 08036 Barcelona (ES); Fundació Clínic per a la Recerca Biomèdica, 08036 Barcelona (ES)
(72) Inventor: ALMENDRO NAVARRO, Vanessa, E-08400 Granollers (ES); GASCÓN VILAPLANA, Pere, E-08036 Barcelona (ES)
(74) Representative: ZBM Patents
(86) International application number: PCT/ES2009/000191
(87) International publication number: WO 2009/125039

(56) References cited:
- WO-A2-03/102136
- WO-A2-03/102136
- US-B1- 7 101 547
- G. RAO ET AL.: "Facilitating role of preprotachykinin-I gene in the integration of breast cancer cells within the stromal compartment of the bone marrow: a model of early cancer progression", CANCER RES., vol. 64, 15 April 2004 (2004-04-15), pages 2874-2881, XP002673519, DOI: 10.1158/0008-5472.CAN-03-3121
- MUÑOZ MIGUEL ET AL: "NK-1 receptor antagonists induce apoptosis and counteract substance P-related mitogenesis in human laryngeal cancer cell line HEp-2.", INVESTIGATIONAL NEW DRUGS, vol. 26, no. 2, 29 September 2007 (2007-09-29), pages 111-118, XP002673520, ISSN: 0167-6997
- RAO G ET AL.: 'Facilitating role of Preprotachykinin-I Gene in the integration of Breast Cancer Cells within the Stromal Compartment of the bone Marrow: A modthe of Early Cancer Progression.' CANCER RESEARCH. vol. 64, no. 8, April 2004, pages 2874 - 2881, XP008145124

### SEQUENCE LISTING

<110> Universidad de Barcelona
   Hospital Clinic i Provincial de Barcelona
   Fundació Clinic per a la Recerca Biomèdica
<120> Antibodies for the treatment of cancer
<130> WO AVCRI028
<150> ES200801071
   <151> 2008-04-09
<160> 9
<170> PatentIn version 3.3
<210> 1
   <211> 11
   <212> PRT
   <213> Homo sapiens
<400> 1
   Arg Pro Lys Pro Gln Gln Phe Phe Gly Leu Met
   1               5                   10
<210> 2
   <211> 7
   <212> PRT
   <213> Artificial
<220>
   <223> fragment 5-11 of substance P amino acid sequence
<400> 2
   Gln Gln Phe Phe Gly Leu Met
   1               5
<210> 3
   <211> 4
   <212> PRT
   <213> Artificial
<220>
   <223> fragment 1-4 of substance P amino acid sequence
<400> 3
   Arg Pro Lys Pro
   1
<210> 4
   <211> 11
   <212> PRT
   <213> Artificial
<220>
   <223> D antagonist
<220>
   <221> D-aminoacid
   <222> (1)..(1)
   <223> D-Arg
<220>
   <221> D-aminoacid
   <222> (5)..(5)
   <223> D-Phe
<220>
   <221> D-aminoacid
   <222> (7)..(7)
   <223> D-Trp
<220>
   <221> D-aminoacid
   <222> (9)..(9)
   <223> D-Trp
<220>
   <221> D-aminoacid
   <222> (11)..(11)
   <223> D-Leu
<400> 4
   Arg Pro Lys Pro Phe Gln Trp Phe Trp Leu Leu
   1               5                   10
<210> 5
   <211> 11
   <212> PRT
   <213> Artificial
<220>
   <223> spantide I
<220>
   <221> D-aminoacid
   <222> (1)..(1)
   <223> D-Arg
<220>
   <221> D-aminoacid
   <222> (7)..(7)
   <223> D-Trp
<220>
   <221> D-aminoacid
   <222> (9)..(9)
   <223> D-Trp
<220>
   <221> D-aminoacid
   <222> (11)..(11)
   <223> D-Leu
<400> 5
   Arg Pro Lys Pro Gln Gln Trp Phe Trp Leu Leu
   1               5                   10
<210> 6
   <211> 20
   <212> DNA
   <213> Artificial Sequence
<220>
   <223> forward primer
<400> 6
   ctacatacac agtggccaag 20
<210> 7
   <211> 20
   <212> DNA
   <213> Artificial Sequence
<220>
   <223> reverse primer
<400> 7
   aatcagtcta ctccgggctc 20
<210> 8
   <211> 19
   <212> DNA
   <213> Artificial Sequence
<220>
   <223> forward primer
<400> 8
   cttcaagcat gccttccgg 19
<210> 9
   <211> 18
   <212> DNA
   <213> Artificial Sequence
<220>
   <223> reverse primer
<400> 9
   cttggggccg tcctctgg 18

## Description

This invention relates to the field of medicine, and particularly to the field of oncology. It specifically relates to therapeutic antibodies for the manufacture of medicaments for the treatment of cancer.

### BACKGROUND ART

Treatment of cancer is a double-edged sword: it should be as aggressive as possible to completely destroy the tumor, but it is precisely this aggressiveness which often causes severe side effects, a reason why some promising therapeutics can not be applied systematically.

Cancer is currently challenged by different and usually combined approaches that can be classified in the following main groups: biological therapies, chemotherapy, hormonal therapies, radiotherapy, stem cell and bone marrow transplants, surgery, and supportive therapies (e.g. bisphosphonates, erythropoietin, hematopoietic growth factors, steroids and platelet transfusions). Biological therapies, also known as immunotherapy, use substances that occur naturally in the body to destroy cancer cells. There are several types of treatment including monoclonal antibodies, cancer growth inhibitors, angiogenesis inhibitors, interferon therapy, vaccines and gene therapy. Immunotherapy directs to a variety of targets.

Many molecular pathways, characteristics of different tumor entities and the physiology of neoplasic cells have been unraveled during recent decades. This knowledge has permitted the design of new drugs to block the action of different proteins involved in the different signal transduction pathways critical for growth and cell division. Among the best characterized pathways there are those mediated by growth factors receptors (e.g. ErbB family). Other families of membrane receptors and thus attractive molecular targets are c-kit, PDGFR, FGFR and VEGFR (platelet-derived, fibroblast, and vascular endothelial growth factor receptors respectively). Tyrosine-kinase (TK) activity inhibitors have been developed for those targets (e.g. gefitinib, erlotinib, and lapatinib), as well as monoclonal antibodies against the extracellular receptor domain. Among the therapeutic monoclonal antibodies approved for use in oncology, trastuzumab is an anti-ErbB2/HER2 for breast cancer, cetuximab is an anti-ErbB1/EGFR for colorectal cancer, and bevacizumab is and anti-VEGF for colorectal, breast and lung cancers (cf. G. Adams et al., "Monoclonal antibody therapy of cancer", Nature Biotechnology 2005, vol. 23, pp. 1147-57). Multitarget inhibitors (such as Sutent) which inhibits TK activity of VEGFR, PDGFR and FGFR, are emerging as promising therapies.

However, despite the major advances in the treatment of cancer due to the introduction of new chemotherapeutic and immunotherapy agents and the optimization of combined therapies, the results are still modest both in the locally advanced disease and in the metastatic stage. Thus, the development of new strategies which ameliorate patient prognostic is necessary. This entails firstly the identification of new functional targets and epitopes on existing targets on the wide range of cancers.

Rao et al (2004. Cancer Res, 64, 2874-2881) discloses treatment of (breast) cancer using a neurotransmitter encoding gene, encoding i.a. substance P, as a target. The authors show the inhibition of the gene TAC1 (herein called PPT-I) coding i.a. for the substance P, with siRNA.

Munoz et al (2008. Invest New Drugs, 26, 111-118) shows that NK-1 receptor antagonists induce apoptosis in laryngeal cancer.

WO03102136 proposes to use antibodies binding to neurokinin B, a family member of substance P, for treatment of many diseases, including cancer.

### SUMMARY OF THE INVENTION

In its broadest sense, the invention relates to subject-matter as defined in the appended claims.

Inventors have surprisingly found that the direct administration of an antibody against substance P to different tumoral cell lines reduces tumoral cell proliferation and induces tumoral cell death. Thus, the present invention relates to the use of an antibody, specific against substance P, for the manufacture of a medicament for therapeutic treatment of a cancer expressing NK1 receptor and/or an ErbB receptor family member (i.e. the epidermal growth factor receptors family), by direct administration to a mammal including a human.

The disclosure may alternatively be formulated as a method for therapeutic treatment of cancer in a mammal including a human, comprising the administration to said mammal of an effective amount of an antibody or a fragment thereof, against substance P, which inhibits the activity of substance P in the subject.

Substance P (SP, CAS Registry Number 33507-63-0) is an undecapeptide implicated in neurotransmission, vasodilatation and motor response. Substance P has the following amino acid sequence: (C-ter) Arg-Pro-Lys-Pro-Gln-Gln-Phe-Phe-Gly-Leu-Met (N-ter) (SEQ ID NO: 1). Substance P belongs to a family of closely-related peptides known as tachykinins (neurokinins), along with two other related endogenously-released peptides, neurokinin A (NKA) and neurokinin B (NKB). Tachykinis are small peptide neurotransmitters that contribute to the pathophysiology of several human diseases. Human tachykinins are codified by the TAC1 gene, including substance P, NKA and NKB, which interact with a family of G-protein coupled receptors (GPCRs), the tachykinin receptors NK1, NK2 and NK3, respectively. These molecules belong to the neuroimmunoendocrine axis, that when dysfunctional has been associated with cancer. The preferential receptor of substance P is codified by a gene that could generate full-length (NK1-FL) and truncated (NK1-Tr) transcripts, the latest related to the transformation of non-tumorigenic breast cells. In breast cancer, the role of substance P and its receptor in the acquisition of oncogenic properties and in facilitation of bone marrow metastasis has been described. Inhibition of TAC1 expression by small interfering RNA in breast cancer cells (BCCs) shows a loss of tumorigenic phenotype and an inhibition of bone marrow metastasis in nude mice (cf. G. Rao et al., "Facilitating role of preprotachykinin-I gene in the integration of breast cancer cells within the stromal compartment of the bone marrow: a model of early cancer progression" Cancer Res. 2004, vol. 64, pp. 2874-81), while stable transfection of NK1-Tr receptor leads to a transforming phenotype (cf. H.J. Patel et al., "Transformation of breast cells by truncated neurokinin-1 receptor is secondary to activation by preprotachykinin-A peptides", Proc Natl Acad Sci USA 2005, vol. 102, pp. 17436-41). Moreover, the expression of NK1-Tr in breast cancer cells stimulates substance P secretion, leading to an autocrine loop formation where receptor and ligand are each other regulated. Previous studies demonstrated the important role of substance P in cancer and, particularly inducing proliferation of BCCs, in the integration of BCCs on bone marrow as well as its function as a stimulator of bone metastasis (cf. A.S. Singh et al., "Oncogenic and metastatic properties of preprotachykinin-I and neurokinin-1 genes" Vascul. Pharmacol. 2006, vol. 45, pp. 235-42; H.S. Oh et al., "Bone marrow stroma influences transforming growth factor-beta production in breast cancer cells to regulate c-myc activation of the preprotachykinin-I gene in breast cancer cells" Cancer Res. 2004, vol. 64, pp. 6327-36). It has been also demonstrated the oncogenic properties of substance P and its high affinity receptor NK1 (cf. H.J. Patel et al., 2005 supra) by its ability to transform non-tumoral breast epithelial cell lines.

According to this invention, direct administration of an antibody against substance P inhibits HER2 activation and expression in a panel of BCCs. This inhibition of HER2 causes a downregulation of different proteins involved in cell cycle progression, like cyclin D1, cyclin E, cdk4 and cdk2. As a consequence, a decrease in proliferation and an arrest in G₁ phase of cell cycle are observed. On the other hand, long term exposure of BCCs to the antibody causes an induction of apoptosis, as analyzed by DAPI nuclei staining, annexin V/PI staining and by the fact that cancer cells detach from the dish 30-48 hours after treatment. These effects are not observed in a transformed non-tumorigenic cell line, which does not detach from the dish neither was affected by antibody treatment, a fact that points to a specific effect of the antibody in tumoral cell lines. The effects of antibody treatment are not-HER2 dependent, although its effects seem to be more pronounced in HER2 overexpressing cell lines than in others with HER2 low expression. Furthermore, in a BC cell line with EGFR overexpression (MD-MBA-468), substance P induces its phosphorylation as well, while antibody treatment strongly inhibits its activity, leading to cell death. Moreover, these antitumor effects are not restricted to BCCs since the treatment of colon cancer or prostate cancer cell lines with the antibody also induce cell death and inhibits HER2 or EGFR basal activation in these cell lines.

In this invention, the term "direct administration" means that the treatment is not made by means of a vaccination with an immunogenic form of substance P for the production of the antibodies by the patient himself. In the present invention, the antibody is produced (out of the body) and it is administered to the patient.

The treatment proposed by the present disclosure is useful to feasible patients (patients with ECOG status from 0 to 2) in a neoadjuvant treatment (treatment previous to surgery), in an adjuvant treatment (complementary treatment after surgery, when no macroscopic tumor is detected) and in a metastatic treatment. The ECOG status from the Eastern Cooperative Oncology Group is a scale used by doctors and researchers to assess the physical condition of the patient, how patient's disease is progressing, how the disease affects the daily living abilities of the patient and determine appropriate treatment and prognosis.

By "cancer" it is understood a malignant tumor of potentially unlimited growth that expands locally by invasion and systemically by metastasis. According to the invention, the antibody or the fragment thereof is administered to subjects with a cancer which expresses the NK1 receptor and/or a ErbB receptor family member. Members of the ErbB receptors family are HER1, HER2, HER3 and HER4 receptors.

A member of the ErbB receptor family with relevant functions in cancer is HER2. Its relation and significance in tumor progression has been described for breast, prostate, lung, colon and other cancer types. In breast cancer, approximately 20% of patients show HER2 overexpression and gene amplification, related to a more aggressive phenotype and poor prognosis.

In a particular embodiment, the cancer (the tumoral cells) expresses NK1 receptor and/or HER1 receptor. In another embodiment the cancer expresses NK1 receptor and/or HER2/neu receptor, and particularly, the cancer expresses NK1 receptor and HER2/neu receptor. In another embodiment, the cancer expresses NK1 receptor and/or HER3 receptor; and in another embodiment, the cancer expresses NK1 receptor and/or HER4 receptor. In a more particular embodiment, the cancer expresses NK1 receptor, HER1 receptor and HER2/neu receptor. As it is shown by the experiments, the effect of the antibody is observed in tumoral cell lines overexpressing HER2 (BT474, SKBR3, MDA-MB-453) but also in tumoral cell lines with a HER2 lower expression. The expression of NK1, HER1, HER2, HER3 and HER4 receptors can be determined in a patient by any method known in the art, such as immunohistochemistry or PCR.

As used in the art, all the terms "c-erb B2", "ErbB2", "EGFR2" and "HER2/neu" refer to the same member 2 of the epidermal growth factor receptors family. "ErbB1", "HER1", "EGFR" and "EGFR1" all refer to member 1 of the family. "HER3" and "ErbB3" correspond to the member 3 of the family and "HER4" and "ErbB4" to the member 4 of the family.

In an embodiment of the invention, the cancer is selected from the group consisting of astrocitoma, glioma, neuroblastoma, pancreatic cancer, melanoma, ovarian cancer, thyroid cancer, pituitary adenoma, prostate cancer, colon cancer and breast cancer. In a particular embodiment, the cancer is breast cancer.

Since the implication of mammalian tachykinins in human diseases was discovered, pharmaceutical industry has been hardly searching for the development of potent antagonists of NK receptors. Although, only one drug has been approved by the FDA for the treatment of chemotherapy induced nausea (aprepitan), different NK1 antagonists are under clinical trials for the treatment of different diseases. The different approaches developed until now have been focused in the inhibition of the receptor and thus, in the development of compounds that compete with substance P for the union with its receptor. It has been suggested in different studies that NK1 receptor antagonists might be useful in anti-cancer therapy (c.f. WO 2001001922 and EP 773026). In studies with 1 nM of NK1 specific antagonist CP-96341-1 (Pfizer) or NK2 specific antagonist SR 48968 (Sanofi), they induced a 60% reduction of BCC colony formation, while the use of both antagonists resulted in a 80% reduction, suggesting that the effects of each antagonist may be additive. But the effects of these antagonists were not as a consequence of apoptosis or necrosis induction, because the cells were 99% viable (cf. D. Singh et al., "Increased expression of preprotachykinin-I and neurokinin receptors in human breast cancer cells: implications for bone marrow metastasis", PNAS 2000, vol. 97, pp. 388-93). Taking together, these experiments pointed to a blockade of cell proliferation by the inhibition of NK receptors rather than an induction of cell death by suppression of NKR signal transduction pathways. In another study also with a human breast cancer model, the NK1 antagonist MEN 11467 and the NK2 antagonist MEN 11420 (nepadutant) induced an inhibition of proliferation (i.e. a cytostatic and not a cytotoxic effect) against the tumor cell line MDA-MB-231 (cf. M. Bigioni et al., "Role of NK-1 and NK-2 tachykinin receptor antagonism on the growth of human breast carcinoma cell line MDA-MB-231 ", Anti-cancer drugs 2005, vol. 16, pp. 1083-9). In the present invention, the inhibition of substance P by an antibody is more potent than in the aforementioned cases in blocking signal transduction through its receptor, since long inhibition of substance P by antibody leads to cell death.

According to the present invention, the treatment of cancer is based on a direct administration of an antibody or a fragment thereof, against substance P, and the effects seen with this treatment are different from other therapies related to substance P known in the art. Inventors have observed that anti-SP antibody administration inhibits RTKs (receptor tyrosine kinase) family members. Therefore, the antibody administration regulates cell proliferation and survival in part by inhibiting RTKs activation.

Moreover, the effects in RTKs such as HER2 or HER1 by the administration of an antibody against substance P are through an intracellular pathway contrary to the action of other antibodies that bind to extracellular domains of receptors, such as trastuzumab against HER2 receptor. Many tumors elude anti-HER2 antineoplasic drugs and develop resistance to these drugs to keep on proliferating. Currently, metastasic HER2-positive breast cancers which fail in responding to the standard anti-HER2 therapy (trastuzumab), find an alternative in lapatinib (TK inhibitor). However it is possible that such tumors succeed in evading to this alternative. Thus, the treatment of the invention is useful in cancers presenting drug resistance to trastuzumab and to other anti-cancer drugs directed to HER2 receptor and HER1.

As used herein, "antibody against substance P" means any polyclonal or monoclonal antibody against substance P. The invention also contemplates the use of fragments of such antibodies. A fragment means a portion of an anti-substance P antibody which is of a sufficient size and conformation to bind to an epitope present on substance P thereby preventing substance P from binding to cellular receptors for substance P. As used herein, the term "specific" means that the antibody has no random associations with other antigens. Numerous well-known assay techniques based upon immunological reactions between antigens and antibodies may be performed using substance P as the antigen to determine whether or not a particular antibody, or antibody fragment, has the ability to bind to an epitope present on the substance P, and to potentially inhibit the activity of substance P. These techniques may be, for example, enzyme-linked immunosorbent assays (ELISA), immunofluorescence assays (IFA), radioimmunoassays, immunoelectrophoresis, and immunoblotting. Moreover, to determine whether a particular antibody or an antibody fragment has the ability to treat cancer, an apoptosis inducing assay can be used, such as DAPI (4'-6-diamidino-2-phenylindole) or annexin staining (see Examples below).

In a preferred embodiment of the invention the antibody or the fragment thereof is against the C-terminal portion of the substance P amino acid sequence from the amino acid 5 to 11, namely, Gln-Gln-Phe-Phe-Gly-Leu-Met (SEQ ID NO: 2).

It is to be understood that the antibody against substance P is in a pharmaceutically acceptable form to be orally, parenterally, intravenously or otherwise administered to a subject. Furthermore, the antibody against substance P of the invention can be administered alone or in a composition with pharmaceutically acceptable carriers or excipients. The person skilled in the art will adapt the composition depending on the particular mode of administration. In the case of administering purified antibody, oral administration is the preferred method and is preferably accomplished through solid dosage forms which include capsules, tablets, pills, powders and granules, among others, or by liquid dosage forms. Preparations of the anti-substance P antibody for parenteral administration preferably include sterile aqueous or non-aqueous solutions, suspensions or emulsions.

The dosage of active ingredient may be selected depending upon the desired therapeutic effect, the route of the administration and the duration of the treatment. Administration dosage and frequency will depend on the size, age and general health condition of the subject, taking into consideration the possibility of side effects. Administration will also be dependent on concurrent treatment with other drugs and subjects' tolerance of the administered drug. Those skilled in the art may ascertain the proper dose using standard procedures. It is understood that the dose should be an effective amount of antibody in the sense that the treatment has at least the same or better effects than current therapies in those patients.

The compositions may comprise the antibody as a single agent against cancer, combinations of such agents, or combinations with other therapeutic agents depending on the condition.

Unless otherwise defined, all technical and scientific terms used herein have the same meaning as commonly understood by a person ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention.

### DESCRIPTION OF THE DRAWINGS

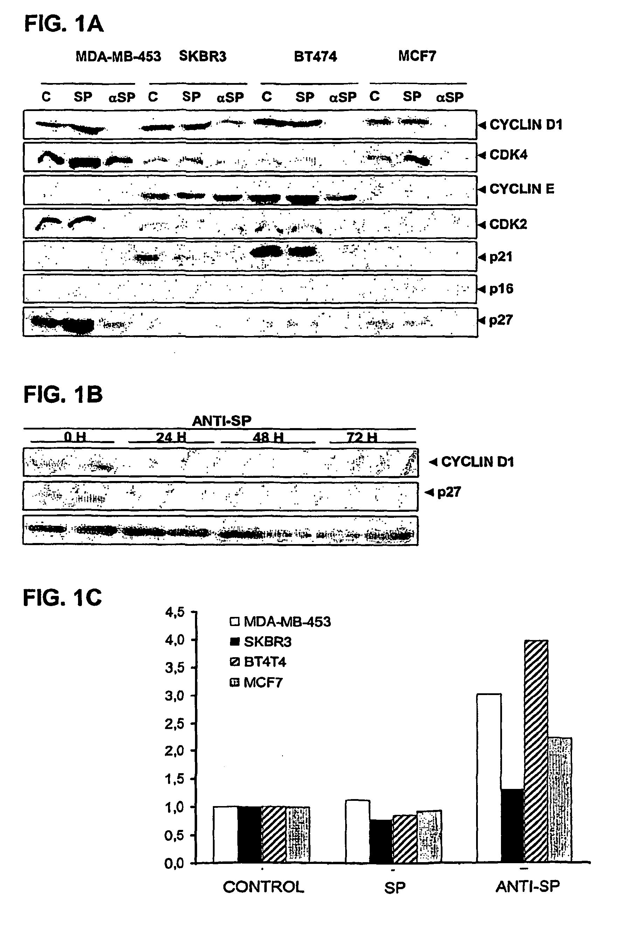

FIG. 1 shows the results of the regulation of cell cycle protein expression by anti-SP antibody treatment. FIG. 1A: For the study of the effect of SP and its inhibition on cell cycle protein expression, cells were treated with 100 nM of SP or antibody anti-SP at 1/100 dilution for 48 hours. The different pattern of protein expression was determined by Western blot. C (control), SP, αSP (anti-SP antibody). FIG. 1 B: For the time-course studies cells were treated at the same concentration of antibody for the times indicated. FIG. 1C: The ratio between p27/cyclin D1 (y-axis) was determined as an indicator of the proliferative rate of the cells.

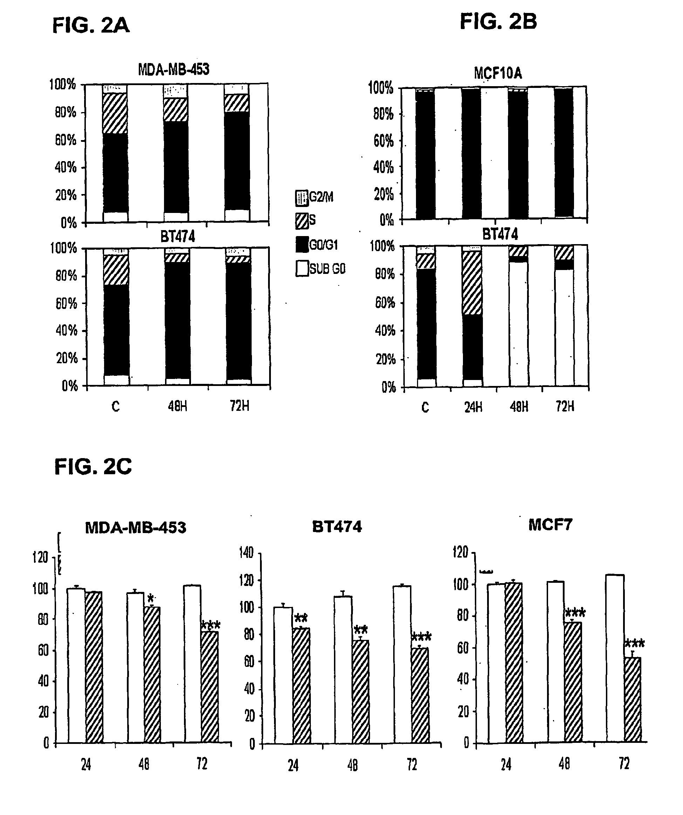

FIG. 2 shows the results of cell cycle distribution of breast cancer cells under antibody treatment. In FIG. 2A, cell cycle analysis was done in subconfluent and in FIG. 2B confluent cells after antibody treatment at the times indicated. In FIG. 2C, proliferation (in %, y-axis) of BCCs under antibody treatment (at different times, in hours, x-axis) was determined by MTS method. Antibody treatment is shown with bars with lines and control with white bars. As it is shown, inhibition of SP by the antibody causes a significant decrease in the proliferation rate of tumoral compared with control cells. The results are mean values ± S.E.M. for 6 samples per group. Statistical significance of the differences (Student's t test): *p<0.05, **p<0.01, ***p<0.001.

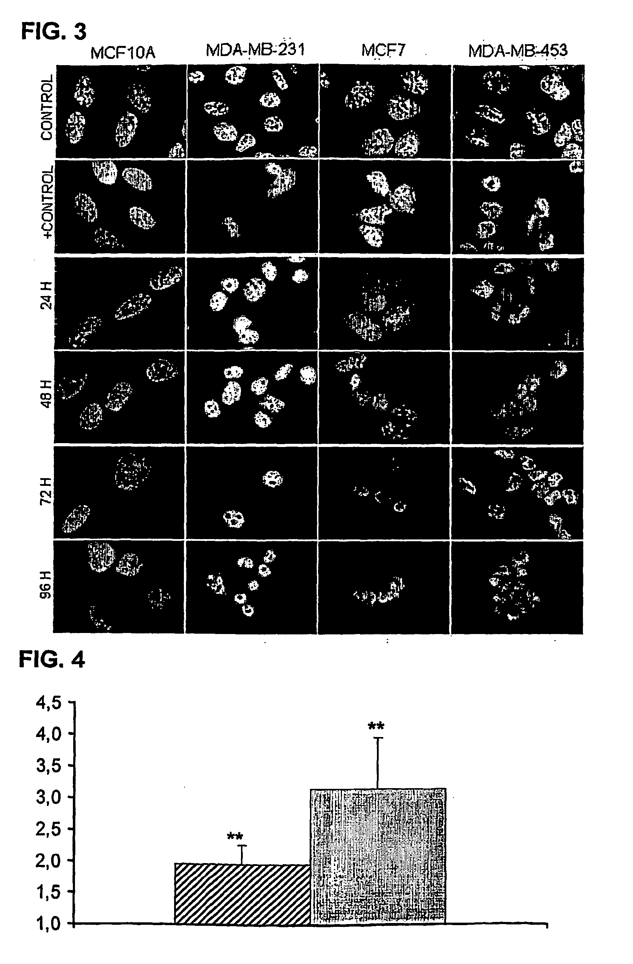

FIG. 3 shows the results of apoptosis induction by anti-SP antibody. Different cell lines were treated at different times with anti-SP antibody at 1/100 dilution. As a positive control for apoptosis induction cells were treated for 24 hours with 100 µM of TNFα. DAPI staining showed a reduction in the nuclei size as well as other apoptotic characteristics like nuclear vacuolization and loss of membrane integrity.
FIG. 4 shows the results of annexin V/PI staining determination on apoptosis induction. Fold apoptosis induction vs. non-treated cells (y-axis) is shown. Bars with lines indicate antibody 1/250 treatment, and grey bars antibody 1 /100 treatment.

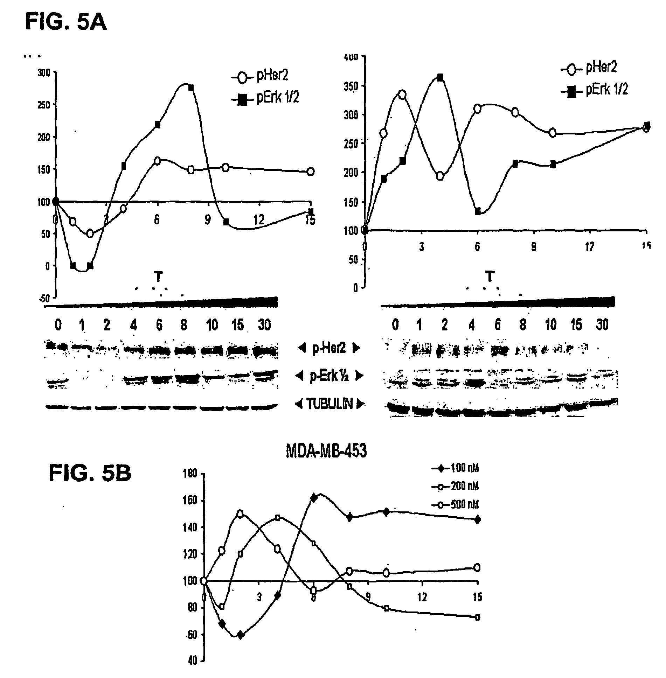

FIG. 5 shows how SP induces transactivation of HER2 in breast cancer cell lines. In FIG. 5A, to analyze the effects of SP on HER2 activation different BC cell lines were treated with SP at 100 nM for the indicated times (in min). Here they are only shown the results for MDA-MB-453 and MDA-MB-231 cells but similar activation points were observed for SKBR3, BT474 and MDA-MB-468 cell lines. y-axis is in arbitrary units. In FIG. 5B, for the dose-response study MDA-MB-453 cell line was treated at different times (minutes, x-axis) with 100, 200 or 500 nM of SP, and then activation of HER2 was determined. The results are expressed as a percentage of control (y-axis), and every experiment was done in duplicate.

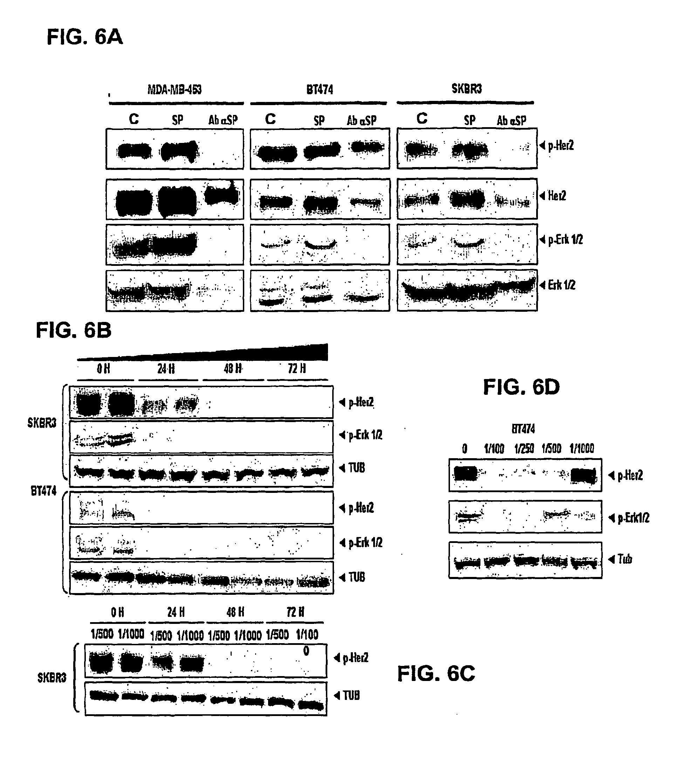

FIG. 6 shows the results of inhibition of HER2 phosphorylation by anti-SP antibodies. In FIG. 6A cells were treated with 500 nM of SP or anti-SP antibody at 1/100 dilution for 48 hours, and the levels of the different proteins determined by Western blot. C means control. Although not shown, to ensure optimal amount of protein load tubulin was used as an internal control. In FIG. 6B for the time-course studies, SKBR3 and BT474 cell lines were treated with anti-SP antibody for 24, 48 and 72 hours and protein analyzed by Western blot. In FIG. 6C for the dose-response studies, SKBR3 was treated with anti-SP antibody for 24, 48 and 72 hours at 1/500 or 1/1000 dilution. Similar pattern on the inhibition of HER2 was observed. FIG. 6D shows a dose-response study in BT474 performed at different dilutions of antibody for 48 hours, and inhibition of HER2 and Erk was observed in a dose-dependent manner.

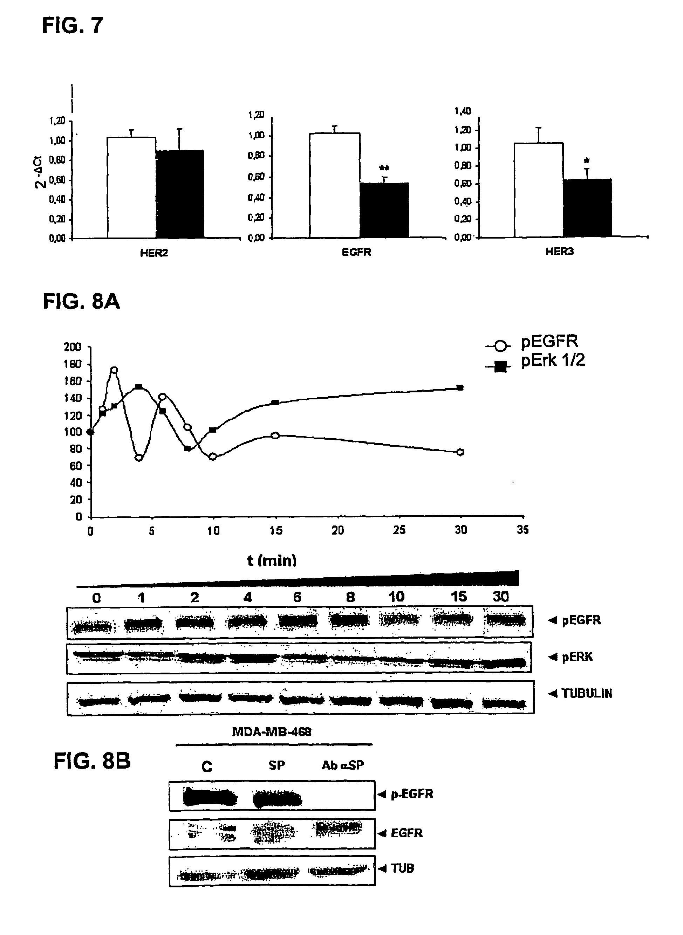

FIG. 7 shows how anti-SP antibody regulates c-erbB genes in MDA-MB-453 cell line. mRNA expression of HER2, EGFR and HER3 was analyzed by real-time PCR. Results are expressed as 2^{-ΔCt} and this indicates the fold induction/decrease of gene expression. White bars indicate control treatment and black bars antibody 1/100 treatment. The results are mean values ± S.E.M. for 3 samples per group. Statistical significance of the differences (Student's t test): *p<0.05, **p<0.01.
FIG. 8 shows the results of EGFR transactivation by SP in MDA-MB-468 cells. FIG. 8A: to analyze the effects of SP on EGFR activation MDA-MB-468 cell line was treated with SP at 100 nM for the indicated times (in min). The levels of phospho-EGFR and phospho-Erk were determined by Western blot analysis. FIG. 8B: The effects of the inhibition of SP in the activation of EGFR were determined as for HER2. C means control.

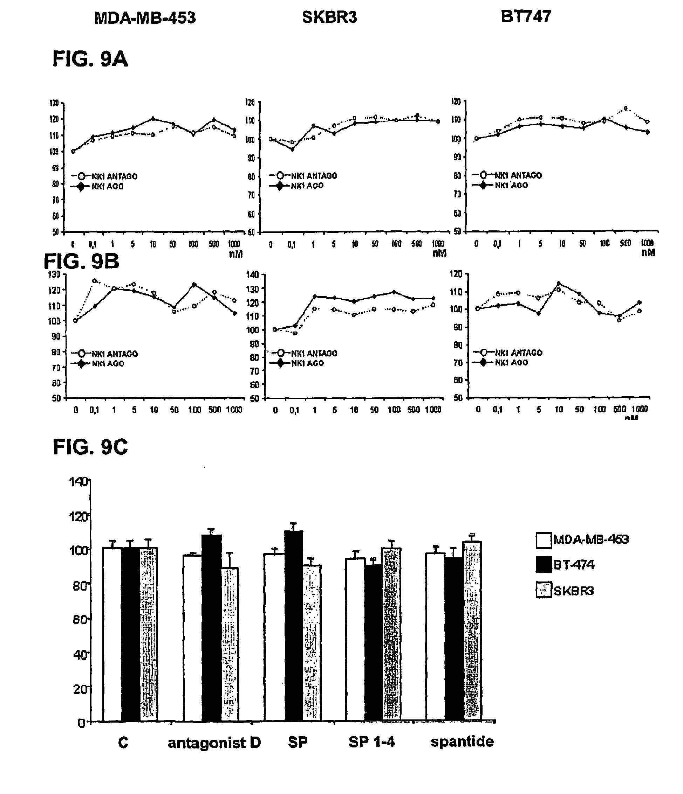

FIG. 9 shows the effects of NK1 antagonists on cell proliferation. To analyze if antagonism of NK1 decrease cell proliferation similar to the use of anti-SP antibodies, cells were treated with agonist (substance P) or antagonist (spantide) of NK1 for 72 hours (FIG. 9A) or every 24 hours for three days (FIG. 9B). Proliferation was determined by MTS assay. In FIG. 9C cells were treated with PBS (control), antagonist D (SEQ ID NO: 4), SP, fragment 1-4 of SP (SEQ ID NO: 3) (agonist) and with spantide (SEQ ID NO: 5), for 72 hours and proliferation rate was assessed by MTS method. C means control.

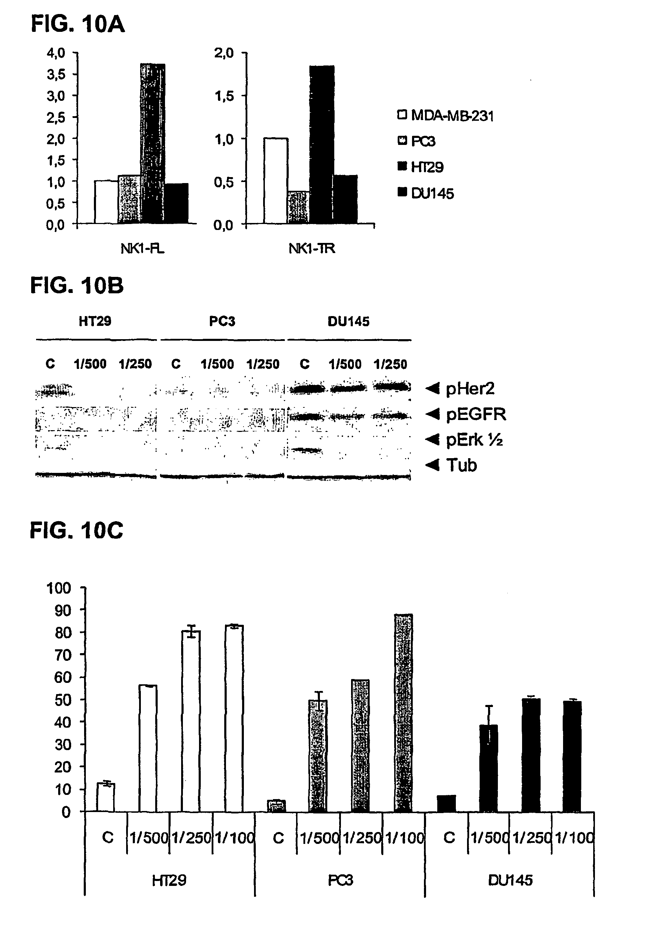

FIG. 10 shows the effects of the anti-SP antibody on colon and prostate cancer cell lines. The colon cancer cell line HT29 and the prostate cancer cell lines PC3 and DU145 express NK1 receptors, as determined by real time-PCR (FIG. 10A). Results are expressed as 2^{-ΔCt} and this indicates the fold induction/decrease of gene expression. The treatment of these cell lines with the anti-SP antibody at 1/500 and 1/250 dilutions inhibited the phosphorylated levels of Her2, EGFR and Erk depending on the cell line (FIG. 10B). Again, the treatment of these cells also induced cell death and increased the population positive for annexin V/IP staining (y-axis) (FIG. 10C). C means control.

### DETAILED DESCRIPTION OF PARTICULAR EMBODIMENTS

### Cell lines

The following cell lines were purchased from American Type Culture Collection and were cultured according to their instructions: MDA-MB-453, metastasic carcinoma from pericardial effusion; BT474, ductal carcinoma; SKBR3, adenocarcinoma from pleural effusion; MDA-MB-231, adenocarcinoma from pleural effusion; MCF7, adenocarcinoma from pleural effusion; MDA-MB-468, adenocarcinoma; MCF10A, normal breast epithelial cells; HT29, colon adenocarcinoma; PC3, bone marrow metastasis from a prostate carcinoma and, Du145, brain metastasis from a prostate carcinoma. All cells were serum starved overnight before experiments if not specified. For some proliferation experiments, cells were under complete growing medium plus fetal, as specified.

### Antibodies and reagents

Antibodies were purchased from different sources: phosphor-EGFR, EGFR, phosphor-p42/44 MAPK, p42/44 MAPK, phosphor-Akt and Akt antibodies were from Cell Signalling; phosphor-HER2 and HER2 antibodies were from Upstate and BioGenex, respectively; cyclin D1, cyclin E, Cdk 2, Cdk 4, p21 were from Santa Cruz, and anti-tubulin antibody from Sigma. Reagents used for cell culture were rhEGF from PreProtech and Insulin was from SIGMA. Substance P (SEQ ID NO: 1), antagonist D ([D-Arg¹, D-Phe⁵, D-Trp^{7,9}, Leu¹¹]-substance P, SEQ ID NO: 4), NK1 antagonist spantide (spantide I, [D-Arg¹, D-Trp^{7,9}, Leu¹¹]-substance P, SEQ ID NO: 5) and fragment 1-4 of substance P (Arg-Pro-Lys-Pro) were all from Sigma. All general reagents were purchased from Sigma, Bio-Rad and Amersham. Rabbit polyclonal antibody against substance P for used in the experiments was purchased from Biogenesis. In some experiments another polyclonal antibody from Biogenex was used.

### Time course studies

For the analysis of substance P treatment on HER2 and EGFR activation, cells were seeded on dishes and growth until 80% confluence. After 24 hours of fetal deprivation, cells were treated at the indicated times with 100 nM SP. Cells were wash two times with cold PBS and rapidly frozen in a -80°C freezer until protein extraction. For the studies of inhibition of substance P, cells were seeded and consider as above. Anti-substance P antibody at 1/100 dilution was added to cells at the indicated times. After treatment, cells not detached were washed twice with PBS and store at -80°C, and cells detached from dishes were centrifuged at 1500 rpm 5 min and the pellets rapidly frozen for protein extraction.

### Cytotoxicity assay

Cell proliferation was assessed in subconfluent cell cultures that had been incubated for 72 hours with anti-substance P antibody or with serial dilutions of agonist and antagonist of NK1. Briefly, cells were seeded in 96-well plates at a density of 10000 cells/well and allowed to attach overnight. After treatment, viability was daily monitored under microscope and cell proliferation was determined with a tetrazolium compound (MTS, Promega) by CellTiter 96 Aqueous One Solution Cell Proliferation Assay Kit, according to the manufacturer's instructions. For color development, 20 µl of MTS were added to each well and then the plate was read on a microplate spectrophotometer (Molecular Dynamics, Sunnyvale, CA) at 490 nm (test wavelength) and 690 nm (reference wavelength). Different doses were assessed in sixtiplicate and every experiment was done in triplicate.

### Western Blot analysis

For protein extraction cells were lysated in ice-cold radioimmunoprecipitation assay buffer (Tris-HCl 50 mM, pH 7.4; NP-40 1 %; Na-deoxycholate, 0.25%, NaCl 150 mM; EDTA 1 mM; PMSF 1 mM; proteinase inhibitors; Na₃VO₄ 1 mM and NaF 1 mM) and sonicated for 10 seconds. After centrifugation (13000 g 5 min) supernatants were quantified for protein content. Equal amounts of proteins were separated by SDS-PAGE and electrophoretically transferred to polyvinylidene difluoride membranes (BioRad Laboratories, Hercules, CA). Membranes were incubated overnight with the corresponding primary antibodies and then one hour with horseradish peroxidase-conjugated secondary antibody (Amersham, Bucks, USA). To confirm equal protein loading membranes were incubated with β-tubulin mouse monoclonal antibody (Sigma). Chemiluminiscence of the bands was detected after ECL treatment (Amersham, Piscataway, NJ) of membranes and subsequent analysis using a Fujifilm LAS3000 imaging system. Different intensities of bands were quantified using Image Gauge software, and correct Mr was compared with pre-stained protein standards (BioRad).

### Apoptosis determination

DAPI staining: Apoptotic morphology was evaluated by fluorescence microscopy following DAPI staining (Roche). Cells were grown on coverslips and treated for the indicated times with 1/100 dilution of anti-substance P antibody. After treatment, coverslips were fixed in cold 95% ethanol and washed with PBS, stained with DAPI, and mounted on slides. Images were captured by using an inverted epifluorescence microscope (Leica). Apoptotic cells were identified by features characteristic of apoptosis (e.g. nuclear condensation, nuclear vacuolization, lost of nuclear membrane integrity and apoptotic bodies).

Annexin V/PI staining: For the analysis of apoptosis induction, cells were seeded on 6-well tissue culture plates and allowed to attach overnight and subsequently cultured in medium containing anti-substance P antibody for additional 72 h. Apoptosis was determined, using Annexin-V-FLUOS Staining Kit (Roche), according to the manufacturer's instructions. Briefly, cells were harvested, blocked for 30 min with 1% of albumin and then incubated for 15 min with Annexin V-FITC and propidium iodide (PI). Controls (binding buffer only, propidium iodide only, and annexin-V only) were run to set appropriate detector gains, compensation, and quadrant gates in the FACSCalibur flow cytometer. Twenty thousand cells for each sample were analyzed by the CellQuest software.

### Quantitative real-time reverse transcription-PCR

Total RNA was isolated by the guanidinium thiocyanate method (Ultraspec RNA, Biotecx Laboratories, Houston, TX), and 1 µg of RNA was reverse transcribed in a final volume of 100 µl using High Capacity cDNA Reverse Transcription Kit (Applied Biosystems) according to the instructions of the manufacturer. Quantitative PCR analysis was done on the ABI PRISM 7700 Sequence Detector System (Applied Biosystems, Foster City, CA). For gene expression determination, Assay-on-demand (Applied Biosystems) was used, which consist of a 6-FAM dye-labeled TaqMan MGB probe and the corresponding unlabeled primers. The PCR reaction mixture consisted of 5 µM of each primer, 10 µl of master mix (TaqMan Universal PCR Master Mix, Applied Biosystems) and 2 µl of cDNA. Transcript levels were normalized to those of beta-actin (Hs99999903_m1) that was used as endogenous control. The levels of HER2, EGFR and HER3 analyzed in triplicate, were calculated using the ΔCt method.

To determine the expression of NK1 FL and NK1Tr receptors a quantitative PCR was done in the iQ5 Real-Time PCR Detection System from BioRad. The primers used were forward: 5' CTACATACACAGTGGCCAAG 3' (SEQ ID NO: 6); and reverse: 5' AATCAGTCTACTCCGGGCTC 3' (SEQ ID NO: 7) for NK1-Tr and forward: 5' CTTCAAGCATGCCTTCCGG 3' (SEQ ID NO: 8); and reverse: 5' CTTGGGGCCGTCCTCTGG 3' (SEQ ID NO: 9) for NK1-FL. Annealing temperature was 60 °C and reaction was performed for 45 cycles. Transcript levels were also normalized to those of beta-actin.

### Cell cycle distribution by flow cytometry analysis

For cell cycle distribution, cells were seeded on 6 cm wells dishes and treated with anti-substance P antibody for the times indicated. Then, cells were trypsinized, incubated for 1 hour with RNAse and stained with propidium iodide at room temperature. Cell cycle was analyzed by a FACSCalibur (Becton-Dickinson) cytometer and results processed by the CellQuest software (Becton-Dickinson).

### Treatment with anti-substance P antibody induces a downregulation of the proteins that control cell cycle progression and alters cell cycle distribution.

Although, not in all cases substance P treatment induces an upregulation of cell cycle proteins expression, in all cases antibody treatment strongly inhibits their expression. These effects were more pronounced in the case of cyclin D1, cdk4, cdk2, p21 and p27 (FIG. 1A). As a consequence, an arrest of cell cycle in G₁ phase was observed (FIG. 2A), which lead to apoptosis in confluent cells (FIG. 2B) as analyzed by the increase observed in the sub-Go fraction. In this case, transformed non-tumorigenic MCF10A cells did not show any changes in the several cell cycle phase proteins neither increase in the sub-Go fraction that correlates with apoptosis.

It has been proposed that the ratio between p27, an inhibitor of cdk2 and cdk4, and cyclin D1 but not their total values, could be used as an indicator of the proliferation status. As it can be observed in FIG. 1C, substance P treatment caused a decrease in p27/cyclin D1 ratio, while its inhibition by the antibody increased it, indicating a lower proliferation rate. In fact, the proliferation of cells treated with the antibody decreased significantly in a dose-dependent manner, as measured by the MTS cell proliferation assay (FIG. 2C).

### Treatment with anti-substance P antibody induces tumoral cell death

With the aim to analyze if tumoral cell death was the result of inhibiting substance P, DAPI nuclei staining was performed in order to assess nuclear condensation and vacuolation, which indicates apoptosis induction. Cells were treated with anti-substance P antibody at 1/100 dilution and nuclei morphology was analyzed every 24 hours for 96 hours. In all tumoral cell lines analyzed, nuclear condensation and vacuolization as well as lost of nuclear membrane integrity was observed at 24 hours, effects that increased in a time-dependent manner (FIG. 2). The induction of apoptosis was observed in HER2 low expression cells (MDA-MB-231 and MCF7) and in a HER2 overexpressing cell line (MDA-MB-453), suggesting that the induction of cell death by the inhibition of substance P is independent on the status of HER2, but dependent on tumoral phenotype since MCF10A epithelial cells were not affected by the treatment (FIG. 3).

Supernatants were analyzed to confirm if the detachment of cells from Petri dishes was caused for other mechanisms than apoptosis. DAPI staining of pellets obtained from medium of cells treated with the antibody showed apoptotic morphology and cellular debris, which indicates that the detachment was not an effect of the antibody in cell adhesion capacity but to induction of cell death.

To further demonstrate that these effects are caused by apoptosis induction, cells treated with the anti-substance P antibody were stained for phosphatidylserine exposure by loading with annexin V. Pl staining was also used to discriminate between necrosis and apoptosis. Antibody treatment at the doses 1/100 and 1/250 induced a significant increase in the percentage of cells positive for annexin V/PI staining, confirming that the cell death induced by inhibition of substance P was an apoptotic process (FIG. 4).

### Treatment with anti-substance P antibody strongly inhibits HER2 activity and expression

Treatment of BBCs with anti-substance P antibody at a 1/100 dilution inhibited HER2 basal phosphorylation as well as its total amount in three different HER2 overexpressing cell lines (FIG. 6A). Moreover, phospho and total p42/44 MAPK (Erk 1/2) in some cases was inhibited as well, as a consequence of HER2 signal transduction inhibition (FIG. 6A). On the other hand, antibody treatment caused the detachment of cells from the Petri dish, a signal of the loss of tumoral phenotype. From these results, anti-substance P antibody could be used as a therapeutic approach to inhibit HER2 and thus, decrease the proliferation rate and the survival of tumoral cells.

To determine if the effects of the antibody were time and dose-dependent, SKBR3 and BT474 were treated for 24, 48 and 72 hours with anti-substance P antibody at 1/100, 1/500 and 1/1000 dilution. As it can be observed in FIG. 6B, HER2 phosphorylation was decreased at 24 hours with a no detection of its phosphorylated form at 72 hours of treatment. Regarding the different doses of antibody, similar effects were observed (FIG. 6C), although cell detachment from dishes were assessed in the 1/1000 dilution from 48 to 72 hours.

To further demonstrate that the effects observed were produced by specific inhibition of substance P, another antibody against substance P from Biogenex was used. In this case a dose-dependent inhibition of phosphor-HER2 in BT474 cells was observed (FIG. 6D).

The effects of antibody treatment on the mRNA content of other c-erbB family members were also analyzed. As it is shown in FIG. 7, anti-substance P antibody treatment induces a significant decrease on the expression of EGFR and HER3, suggesting that the effects of the antibody can also affect gene expression of the c-erbB family.

### Treatment with anti-substance P antibody also induces a downregulation of phospho-EGFR

To check if substance P also induces an activation of EGFR, MDA-MB-468 cells were treated at the indicated times with 100 nM of SP. Activation of EGFR was observed at 2 min, with a second activation point at minute 6 (FIG. 8A). Interestingly, as it was observed for HER2, the treatment with anti-substance P antibody also inhibited the basal levels of phospho-EGFR (FIG. 8B).

### Effects of antagonists or agonists of NK1 on cell proliferation

In order to study whether NK1 antagonism could have the same effects on cell proliferation as the inhibition of substance P, it was determined if the proliferation rate of BCCs was altered by the use of an agonist and an antagonist of NK1: fragment 1-4 of substance P and spantide. As it can be seen in FIG. 9, by MTS assay the proliferation rate of BCCs was not altered by the use of NK1 antagonists neither by the addition of substance P at doses ranged from 0.1 to 1000 nM. These results suggest that at the doses tested: 1) the addition of substance P not always affects cell proliferation, suggesting that the substance P-NK1 pathway may function as a saturated system and, 2) the inhibition of NK1 by antagonist is not a correct approach to analyze its effects in short time studies of cell proliferation. In fact, previous studies where NK1 was inhibited by the use of antagonists were clonogenic assays, which required two weeks of cell growth to report results (cf. G. Rao et al., Cancer Res 2004, supra).

### Effects of the antibody anti-SP in colon and prostate cancer cell lines.

To determine if antibody treatment could induce cell death in cancer cells from other origin, it was determined if colon and prostate cancer cells express NK1 receptors. HT29 (colon cancer) and PC3 and Du145 (prostate cancer) cells express NK1 receptors (full length and truncated isoforms) (FIG. 10A), suggesting that similarly to BCCs, substance P has biological effects in these cells lines. In fact, inhibition of substance P by the anti-SP antibody decreased the phosphorylated levels of Her2, EGFR and Erk1/2 depending on the cell line (FIG. 10B). Similarly to the results observed with BCCs, the anti-SP antibody also induced cell death in the HT29, PC3 and Du145 cancer cells lines, as determined by the staining with annexin V/IP (FIG. 10C). The induction of apoptosis in these cell lines was dose-dependent, as increased amounts of anti-SP antibody increased cell death.

## Claims

1. An antibody specific against substance P for use in a method of therapeutic treatment of a cancer expressing NK1 receptor and/or an ErbB receptor family member, by direct administration to a mammal, including a human.

2. The antibody according to claim 1 for use according to claim 1, wherein the cancer expresses NK1 receptor and/or HER1 receptor.

3. The antibody according to claim 1 for use according to claim 1, wherein the cancer expresses NK1 receptor and/or HER2/neu receptor.

4. The antibody according to claim 3 for use according to claim 3, wherein the cancer expresses NK1 receptor and HER2/neu receptor.

5. The antibody according to claim 1 for use according to claim 3, wherein the cancer expresses NK1 receptor and/or HER3 receptor.

6. The antibody according to claim 1 for use according to claim 3, wherein the cancer expresses NK1 receptor and/or HER4 receptor.

7. The antibody according to claim wherein the cancer expresses NK1 receptor, HER1 receptor and HER2/neu receptor.

8. The antibody according to claim 1 for use according to claim 3, wherein the cancer is selected from the group consisting of astrocitoma, glioma, neuroblastoma, pancreatic cancer, melanoma, ovarian cancer, thyroid cancer, pituitary adenoma, prostate cancer, colon cancer and breast cancer.

9. The antibody according to claim 8 for use according to claim 8, wherein the cancer is breast cancer.

10. The antibody according to any of the claims 1-9 for use according to claim 1-9, wherein the antibody recognizes the amino acid sequence SEQ ID NO: 2.

## Patentansprüche

1. Antikörper, der speziphisch gegen den Stoff P ist zur Verwendung in einem Verfahren therapeutischer Behandlung einer Krebserkrankung , die den NK1-Rezeptor und/oder einen Mitglieder der ErbB-Rezeptorfamilie exprimiert durch direkte Verabreichung einem Säugetier, einschließlich eines Menschen.

2. Antikörper nach Anspruch 1 zur Verwendung nach Anspruch 1, wobei die Krebserkrankung den NK1-Rezeptor und/oder den HER1-Rezeptor exprimiert.

3. Antikörper nach Anspruch 1 zur Verwendung nach Anspruch 1, wobei die Krebserkrankung den NK1-Rezeptor und/oder den HER2/neu-Rezeptor exprimiert.

4. Antikörper nach Anspruch 3 zur Verwendung nach Anspruch 3, wobei die Krebserkrankung den NK1-Rezeptor und den HER2/neu-Rezeptor exprimiert.

5. Antikörper nach Anspruch 1 zur Verwendung nach Anspruch 1, wobei die Krebserkrankung den NK1-Rezeptor und/oder den HER3-Rezeptor exprimiert.

6. Antikörper nach Anspruch 1 zur Verwendung nach Anspruch 1, wobei die Krebserkrankung den NK1-Rezeptor und/oder den HER4-Rezeptor exprimiert.

7. Antikörper nach Anspruch 1 zur Verwendung nach Anspruch 1, wobei die Krebserkrankung den NK1-Rezeptor, den HER1-Rezeptor und den HER2/neu-Rezeptor exprimiert.

8. Antikörper nach Anspruch 1 zur Verwendung nach Anspruch 1, wobei die Krebserkrankung ausgewählt ist aus der Gruppe bestehend aus Astrozytom, Gliom, Neuroblastom, Pankreastumoren, Melanom, Eierstockkrebst, Schilddrüsenkrebs, Hypophisenadenom, Prostatakrebs, Dickdarmkrebs und Brustkrebs.

9. Antikörper nach Anspruch 8 zur Verwendung nach Anspruch 8, wobei die Krebserkrankung Brustkrebs ist.

10. Antikörper nach einem der Ansprüche 1-9 zur Verwendung nach Ansprüchen 1-9, wobei der Antikörper die Aminosäuresequenz SEQ ID NO: 2 erkennt.

## Revendications

1. Anticorps spécifique contre une substance P pour utilisation dans un procédé de traitement thérapeutique contre du cancer exprimant le récepteur NK1 et/ou un récepteur de la famille ErbB, moyennant administration directe à un mammifère, y compris les humains.

2. Anticorps selon la revendication 1 pour utilisation selon la revendication 1, dans lequel le cancer exprime le récepteur NK1 et/ou le récepteur HER1.

3. Anticorps selon la revendication 1 pour utilisation selon la revendication 1, dans lequel le cancer exprime le récepteur NK1 et/ou le récepteur HER2/neu.

4. Anticorps selon la revendication 3 pour utilisation selon la revendication 3, dans lequel le cancer exprime le récepteur NK1 et le récepteur HER2/neu.

5. Anticorps selon la revendication 1 pour utilisation selon la revendication 1, dans lequel le cancer exprime le récepteur NK1 et/ou le récepteur HER3.

6. Anticorps selon la revendication 1 pour utilisation selon la revendication 1, dans lequel le cancer exprime le récepteur NK1 et/ou le récepteur HER4.

7. Anticorps selon la revendication 1 pour utilisation selon la revendication 1, dans lequel le cancer exprime le récepteur NK1, le récepteur HER1 et le récepteur HER2/neu.

8. Anticorps selon la revendication 1 pour utilisation selon la revendication 1, dans lequel le cancer est choisi dans le groupe constitué de l'astrocytome, du gliome, du neuroblastome, du cancer pancréatique, du mélanome, du cancer de l'ovaire, du cancer de la thyroïde, de l'adénome de l'hypophyse, du cancer de la prostate, du cancer du côlon et du cancer du sein.

9. Anticorps selon la revendication 8 pour utilisation selon la revendication 8, dans lequel le cancer est du cancer de sein.

10. Anticorps selon l'une quelconque des revendications 1-9 pour utilisation selon les revendications 1-9, dans lequel l'anticorps reconnaît la séquence d'acides aminés SEQ ID NO : 2.
